(19) Europäisches Patentamt
European Patent Office
Office européen des brevets

(11) **EP 4 454 686 A1**

(12) **EUROPEAN PATENT APPLICATION**

(43) Date of publication:
**30.10.2024   Bulletin 2024/44**

(21) Application number: **23170493.3**

(22) Date of filing: **27.04.2023**

(51) International Patent Classification (IPC):
***A61M 5/32*** *(2006.01)*

(52) Cooperative Patent Classification (CPC):
**A61M 5/3202;** A61M 2005/3104

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Becton, Dickinson and Company Franklin Lakes, NJ 07417-1880 (US)**

(72) Inventors:
• **RODRIGUEZ SAN JUAN, Nestor Hamburg, New Jersey 07419 (US)**
• **SINGER, Julien 38420 DOMENE (FR)**

(74) Representative: **Germain Maureau 12, rue Boileau 69006 Lyon (FR)**

(54) **TWO-PART SEALING CAP FOR A SYRINGE TIP**

(57)    A sealing cap for a syringe having a syringe barrel defining a reservoir for containing a medicament and a distal tip having a passageway in fluid communication with the reservoir, the sealing cap including a housing having an open proximal end, a closed distal end, and a sidewall extending from the proximal end to the distal end and defining a cavity for receiving the distal tip, and a sealing insert covering at least a portion of an inner surface of the sidewall of the housing. The material properties of the housing are different from the material properties of the sealing insert, the inner diameter of the sealing insert is less than the outer diameter of at least a portion of the distal tip, and the sealing cap is adapted to cover the passageway and create a seal between the sealing insert and an outer surface of the distal tip.

EP 4 454 686 A1

**Description**

BACKGROUND OF THE INVENTION

Field of the Invention

The present invention is directed to a two-part sealing cap for covering the tip of a syringe to keep contaminants from contacting the tip of the syringe and/or entering the fluid passageway in the tip of the syringe and/or a needle extending from the tip of the syringe, thereby potentially contaminating the medicament contained in the syringe.

Description of Related Art

For prefilled syringes, the avoidance of the contamination of the syringe tip, needle, and/or contents of the syringe is imparted by a cap provided in an interference fit with the tip and covers the tip and/or needle. A seal is formed between the cap and the tip. The seal is necessary for and contributes to the overall container closure integrity (CCI) of the syringe assembly, which is a measurement of the overall capability of the syringe assembly to keep the medicament contained in the syringe from being contaminated.

The cap is generally made of a single material, which is used both for the interface between the cap and the tip and for the application of the necessary contact force to create a seal between the cap and the tip. The cap may be made of an elastomer, for example, a rubber or thermoplastic elastomer, or a plastic, for example, polypropylene (PP).

The material must be sufficiently soft and flexible to ensure a good sealing interface and must also be of sufficient thickness to provide the necessary contact force for sealing. In order to provide the necessary contact force, the flexible material is required to have a substantial thickness, which increases the overall size of the cap. As a result, the diameter of the cap may be larger than the diameter of the syringe barrel, thereby increasing the packaging and storage space required for the syringe assembly. Further, using a single material for the cap requires a trade-off between the softness and flexibility required for the sealing interface and the stiffness required to provide the necessary contact force. Therefore, there is a need for a sealing cap for a syringe that improves the sealing between the syringe tip and the cap to improve the overall CCI of the syringe assembly while reducing the overall size of the cap.

SUMMARY OF THE INVENTION

The present invention is directed to a sealing cap for a syringe comprising a syringe barrel defining a reservoir for containing a medicament and a distal tip having a passageway in fluid communication with the reservoir. The sealing cap comprises a housing having an open proximal end, a closed distal end, and a sidewall extending from the proximal end to the distal end and defining a cavity for receiving the distal tip, and a sealing insert covering at least a portion of an inner surface of the sidewall of the housing. The material properties of the housing are different from the material properties of the sealing insert, the inner diameter of the sealing insert is less than the outer diameter of at least a portion of the distal tip, and the sealing cap is adapted to cover a distal end of the passageway and create a seal between the sealing insert and an outer surface of the distal tip to keep air and contaminants from entering the cavity of the sealing cap and/or the passageway.

The sealing insert may extend from the proximal end of the housing to the distal end of the housing, thereby covering the entire inner surface of the sidewall of the housing or may cover a proximal portion of the inner surface of the sidewall of the housing leaving a distal portion of the inner surface of the housing uncovered. The sealing insert may be a sleeve that is concentric with the sidewall of the housing.

The material of the housing may have a higher Young's modulus, higher strength, higher hardness, and/or lower elasticity than the material of the sealing insert. The sealing insert may be made from any polymer material softer than the housing which at the contact pressures developed at the interface will be operating within an elastoplastic region of the material, for example, ± 20% yield strength in the case of a polymeric sealing layer, for example, the sealing insert may be made from a material selected from the group consisting of polyethylene (PE), polypropylene (PP), and elastomers including thermoplastic, thermo-vulcanizable, or thermoset rubbers, and the housing may be made from a material that is stiffer than the sealing insert, for example, a material selected from the group consisting of polycarbonate (PC), polyoxymethylene (POM), polybutylene terephthalate (PBT), fiber-reinforced polymer, and metal.

The radial thickness of the sidewall of the sealing insert may be greater than a surface roughness of the outer surface of the distal tip, and may be at least 20% thicker than the rms surface roughness (measured such as to cover the roughness scale between 2 nm and 0.1 $\mu$m) of the outer surface of the distal tip.

The sealing cap provides a contact pressure between the sealing insert and the outer surface of the distal tip of 0.1-50 MPa depending on the type of sealing layer implemented (elastomeric to polymeric type correspondently).

The radial thickness of the sidewall of the sealing insert may be minimized while being greater than a surface roughness of the outer surface of the distal tip, and a radial thickness of the sidewall of the housing may be set to a thickness that provides enough stiffness to develop the desired contact pressure at the sealing insert outer surface without significant flexural deformation of sidewall housing to produce the desired strength without undesired geometrical effects, such as significant increase in the outer diameter of the housing. The radial thickness of the sidewall of the housing may be approximately equal to the radial thickness of a cap constructed completely from the material of the insert that provides the contact pressure multiplied by the Young's modulus of the insert material divided by the Young's modulus of the housing material, such that the combination of the Young's modulus of the housing material and the radial thickness of the sidewall of the housing provides the sealing cap with the same contact force as the cap constructed completely of the insert material.

The housing may include one or more recesses where a portion or all of the radial thickness of the sidewall of the housing has been removed. Any recesses extending completely through the radial thickness of the sidewall of the housing may be completely covered by the sealing insert. A plurality of recesses may be provided around a circumference of the housing, such that material left between the recesses comprises a plurality of legs extending from a proximal portion of the housing to a distal portion of the housing.

The sealing insert may have one or more circumferential ribs extending radially inward or the housing may have one or more circumferential ribs extending radially inward from the sidewall of the housing with the contour of the sealing insert following the contour of the housing.

The sealing cap may be adapted to cover a needle extending from the distal tip.

The present invention is also directed to a syringe comprising a syringe barrel defining a reservoir for containing a medicament, a distal tip having a passageway in fluid communication with the reservoir, and a sealing cap as described above, wherein the sealing cap covers a distal end of the passageway and creates a seal between the sealing insert and an outer surface of the distal tip to keep air and contaminants from entering the cavity of the sealing cap and/or the passageway.

The syringe may further comprise a needle extending from the distal tip, and the sealing cap may be adapted to cover the needle.

The outer surface of the distal tip may be substantially cylindrical or may taper such that the outer diameter of the proximal end of the distal tip adj acent the distal end of the syringe barrel is larger than the outer diameter of the distal end of the distal tip. Circumferential ribs extend from an outer surface of the distal tip and contact the sealing insert.

BRIEF DESCRIPTION OF THE DRAWINGS

FIG. 1 is a cross-sectional view of a sealing cap according to the invention placed on a syringe having a cylindrical distal tip;

FIG. 2 is a cross-sectional view of a sealing cap according to the invention placed on a syringe having a cylindrical distal tip and a needle;

FIG. 3 is a cross-sectional view of a sealing cap according to the invention placed on a syringe having a tapered distal tip;

FIG. 4 is a cross-sectional view of a sealing cap according to the invention placed on a syringe having a tapered distal tip and a needle;

FIG. 5 is a cross-sectional view of a sealing cap according to the invention;

FIG. 6 is a cross-sectional view of an alternative sealing cap according to the invention;

FIG. 7 is a side view of a sealing cap according to the invention in which the housing has recesses;

FIG. 8 is a cross-sectional view of a sealing cap according to the invention that includes ribs extending from the sealing insert;

FIG. 9 is a cross-sectional view of a sealing cap according to the invention that includes ribs extending from the housing;

FIG. 10 is a cross-sectional view of a sealing cap according to the invention placed on the distal tip of a syringe, where the distal tip of the syringe has ribs; and

FIG. 11 is a cross-sectional view of a sealing cap according to the invention in which the insert is provided in a recess in the housing.

DESCRIPTION OF THE INVENTION

As used herein, any numerical values are expressed using a period as a decimal point and a comma as a thousand separator, for example, 1,234 would be one thousand two hundred thirty four, and 1.2 would be one and two tenths. Unless otherwise expressly specified, all numbers, such as those expressing values, ranges, amounts or percentages, may be read as if prefaced by the word "about", even if the term does not expressly appear. Any numerical range recited herein is intended to include all sub-ranges subsumed therein. For example, a range of "1 to 10" is intended to include any and all sub-ranges between and including the recited minimum value of 1 and the recited maximum value of 10, that is, all subranges beginning with a minimum value equal to or greater than 1 and ending with a maximum value equal to or less than 10, and all subranges in between, e.g., 1 to 6.3, or 5.5 to 10, or 2.7 to 6.1. Plural encompasses singular and vice versa. When ranges are given, any endpoints of those ranges and/or numbers within those ranges can be combined with the scope of the present invention. "Including", "such as", "for example" and like terms mean "including/such as/for example but not limited to".

For purposes of the description hereinafter, spatial orientation terms, as used, shall relate to the referenced embodiment as it is oriented in the accompanying drawings, figures, or otherwise described in the following detailed description. However, it is to be understood that the embodiments described hereinafter may assume many alternative variations and configurations. It is also to be understood that the specific components, devices, features, and operational sequences illustrated in the accompanying drawings, figures, or otherwise described herein are simply exemplary and should not be considered as limiting.

The present invention is directed to a sealing cap 10 for a syringe 12 and a syringe 12 including such a sealing cap 10.

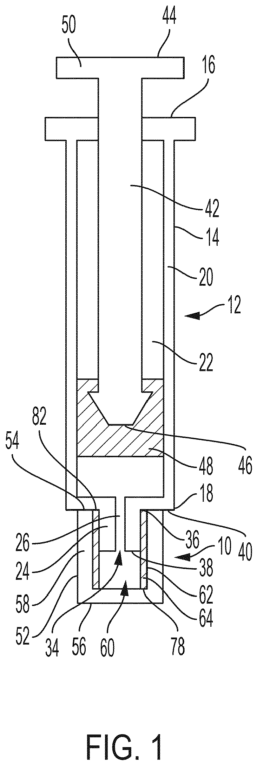

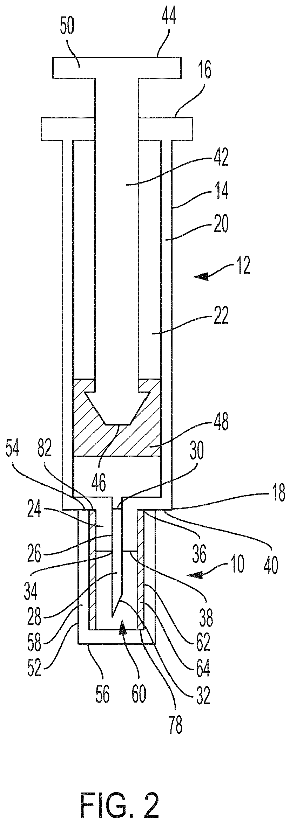

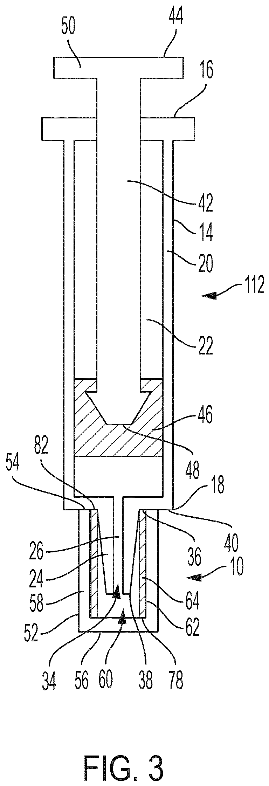

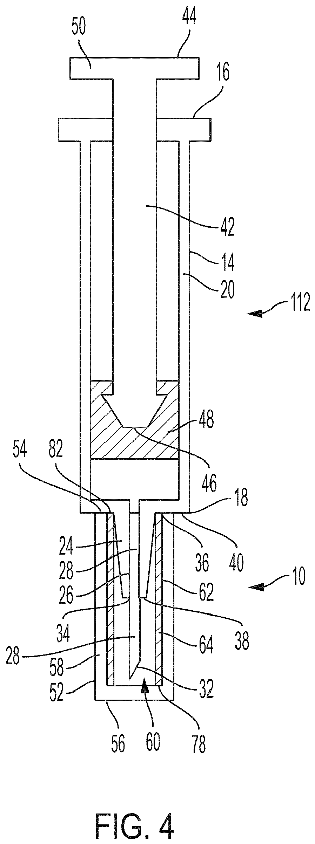

As shown in FIGS 1-4, the inventive sealing cap 10 is adapted for use with a syringe 12 comprising a syringe barrel 14 having a proximal end 16, a distal end 18, and a sidewall 20 extending from the proximal end 16 to the distal end 18 and defining a reservoir 22 for containing a medicament. A distal tip 24 of the syringe 12 extends from the distal end 18 of the syringe barrel 14. A fluid passageway 26 extends through the distal tip 24 of the syringe 12 through which the medicament may be ejected from the reservoir 22.

Optionally, a needle 28 having a proximal end 30 and a pointed distal end 32 may be attached to the distal tip 24 of the syringe 12 such that the needle 28 is in fluid communication with the fluid passageway 26 and/or the reservoir 22. For example, the needle 28 may be inserted through the fluid passageway 26 such that the proximal end 30 of the needle 28 is disposed within the fluid passageway 26, the needle 28 may be partially inserted into the fluid passageway 26, or the needle 28 may be attached to a distal end 34 of the fluid passageway 26.

Alternatively, the distal tip 24 of the syringe 12 may be a luer lock for connecting the syringe barrel 14 to another medical device, such as a needle hub, an intravenous catheter, or the like.

The distal tip 24 of the syringe 12 may have an outer surface that is substantially cylindrical or the distal tip 24 of the syringe 112 may taper such that an outer diameter of the proximal end 36 of the distal tip 24 of the syringe 112 adjacent the distal end 18 of the syringe barrel 14 is larger than an outer diameter of the distal end 38 of the distal tip 24 of the syringe 12. The largest outer diameter of the distal tip 24 of the syringe 12, 112 is smaller than an outer diameter of the syringe barrel 14, thereby forming a distal face 40 at the distal end 18 of the syringe barrel 14.

The syringe 12 may include a plunger rod 42 having proximal end 44 and a distal end 46 with a stopper 48 extending from the distal end 46 and a pressing surface 50 extending from the proximal end 44. The stopper 48 forms a seal with the sidewall 20 of the syringe barrel 14 to contain the medicament within the reservoir 22, and the plunger rod 42 is moveable within the reservoir 22 of the syringe barrel 14 to eject the medicament from the reservoir 22 through the fluid passageway 26 and/or the needle 28.

The inventive sealing cap 10 covers the distal end 34 of the fluid passageway 26 and/or the distal end 32 of the needle 28 and creates a seal with the distal tip 24 of the syringe 12 to keep air and contaminants from entering the distal end 34 of the fluid passageway 26 and/or the distal end 32 of the needle 28 and thereby contaminate the medicament.

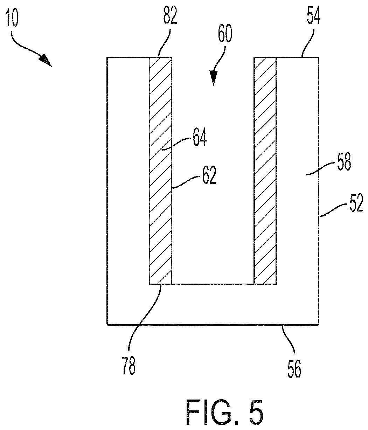

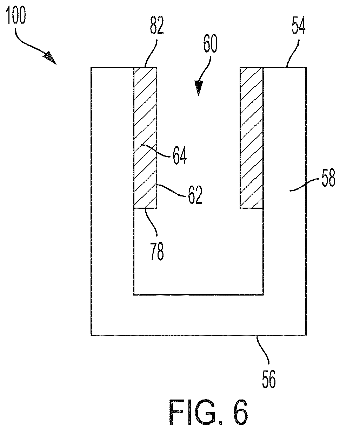

The sealing cap 10 comprises a housing 52 having an open proximal end 54, a closed distal end 56, and a sidewall 58 extending from the proximal end 54 to the distal end 56 and defining a cavity 60 for receiving the distal tip 24 of the syringe 12 of the syringe barrel 14 and, optionally, the needle 28. A sealing insert 62 covers at least a portion of the inner surface of the sidewall 58 of the housing 52. The sealing insert 62 may extend from the proximal end 54 of the housing 52 to the distal end 56 of the housing, thereby covering the entire inner surface of the sidewall 58 of the housing 52 (FIG. 5). In another embodiment, the sealing insert 62 of the sealing cap 100 may only cover a proximal portion of the inner surface of the sidewall 58 of the housing 52 leaving a distal portion of the inner surface of the housing 52 uncovered (FIG. 6).

The sealing insert 62 may take the form of a sleeve that is concentric with and lines the inner surface of the sidewall 58 of the housing 52.

An inner diameter of the sealing insert 62 is less than the outer diameter of at least a portion of the distal tip 24 of the syringe 12, such that the sealing insert 62 is compressed and/or the sidewall 58 of the housing is deflected radially outward, thereby creating a frictional fit and a circumferential seal between the sealing insert 62 and the outer surface of the distal tip 24 of the syringe 12. For example, if the distal tip 24 of the syringe 12 is substantially cylindrical, the sealing insert 62 may have an inner diameter that is less than an outer diameter of the distal tip 24 of the syringe 12, if the distal tip 24 of the syringe 12 has a tapered outer surface, the sealing insert 62 may have an inner diameter that is less than the outer diameter of at least a portion of the distal tip 24 of the syringe 12, or if the outer surface of the

EP 4 454 686 A1

distal tip 24 of the syringe 12 includes protrusions or ribs, the insert may have an inner diameter that is less than an outer diameter of the protrusions or ribs or an inner diameter that is less than the outer diameter of the distal tip 24 of the syringe 12 in the areas between the protrusions or ribs.

The material of the housing 52 provides the necessary compressive contact force between the sealing cap 10 and the distal tip 24 of the syringe 12 while the material of sealing insert 62 provides a suitably soft material for creating a frictional fit with a continuous seal between the sealing cap 10 and the distal tip 24 of the syringe 12.

The sealing insert 62 may be made from any suitable material that is sufficiently flexible to compress and conform to the outer surface of the distal tip 24 of the syringe 12 to create a seal between the sealing insert 62 and the outer surface of the distal tip 24 of the syringe 12 that prevents air and contaminants from entering the cavity 60 of the sealing cap 10. The material of the sealing insert 62 may be softer than the material of the housing 52 and may have a lower Young's modulus (stiffness), lower strength, lower hardness, and/or higher elasticity than the material of the housing 52. For example, the sealing insert 62 may be made from a polymer including polyethylene (PE), polypropylene (PP), an elastomer, or a rubber.

The housing 52 may be made from any suitable material that provides sufficient compressive contact force on the sealing insert 62 to maintain the sealing cap 10 on the syringe 12 until the sealing cap 10 is removed by a user and to maintain a continuous seal between the sealing insert 62 and the outer surface of the distal tip 24 of the syringe 12 that prevents air and contaminants from entering the cavity 60 of the sealing cap 10. The material of the housing 52 may be stiffer than the material of the housing 52 and may have a higher Young's modulus (stiffness), higher strength, higher hardness, and/or lower elasticity than the sealing insert 62. For example, the housing 52 may be made from polycarbonate (PC), polyoxymethylene (POM), polybutylene terephthalate (PBT), fiber-reinforced polymer, and/or metal.

The Young's modulus of the sealing insert 62 may be less than 50% of the Young's modulus of the housing 52. The Young's modulus of the sealing insert 62 may be 0.001-0.1 GPa and the Young's modulus of the housing 52 may be 1-10 GPa.

The thickness of the sidewall 64 of the sealing insert 62 in a radial direction is greater than the surface roughness of the outer surface of the distal tip 24 of the syringe 12. The thickness the sidewall 64 of the sealing insert 62 may be at least 20% thicker than the rms surface roughness (measured such as to cover the roughness scale between 2 nm and 0.1 $\mu$m) of the outer surface of the distal tip 24 of the syringe 12 and may be 0.05-1 mm, preferably 100 $\mu$m - 0.5 mm. Below a thickness of 0.1 mm, the thickness of the sidewall 64 of the sealing insert 62 may be too thin to compress sufficiently to form a continuous seal with the distal tip 24 of the syringe 12 and the reduction in the overall size of the sealing cap 10 is negligible. For example, for a distal tip 24 of the syringe 12 made of glass, which has nanometric-scale rms roughness of less than 50-100 nm, the sealing insert 62 may have a thickness as low as 0.05 $\mu$m. For a distal tip 24 of the syringe 12 made of plastic, with micrometer-scale rms roughness of 5-50 $\mu$m, the sealing insert 62 may have a thickness of 7.5-75 $\mu$m.

The housing 52 provides a contact pressure between the sealing insert 62 and the outer surface of the distal tip 24 of the syringe 12 of 0.1-50 MPa. Depending on the Young's modulus (stiffness) of the material of the housing 52, the thickness of the sidewall 58 of the housing 52 in a radial direction may be 10-500 $\mu$m, preferably 50-200 $\mu$m, in order to provide the desired contact force.

In order to decrease the overall size of the sealing cap 10 while providing the desired contact force, the radial thickness of the sidewall 64 of the sealing insert 62 is minimized based on the surface roughness of the outer surface of the distal tip 24 of the syringe 12 as discussed above. The radial thickness of the sidewall 58 of the housing 52 is then set to a thickness that, based on the Young's modulus of the housing material, will provide the desired contact pressure of 0.1-50 MPa between the sealing insert 62 and the outer surface of the distal tip 24 of the syringe 12. The minimum contact pressure of 0.01 MPa provides sufficient frictional force between the sealing insert 62 and the outer surface of the distal tip 24 of the syringe 12 to maintain the sealing cap on the syringe 12 and provides a continuous seal between sealing insert 62 and the outer surface of the distal tip 24 of the syringe 12 to prevent air and contaminants from entering the cavity 60 of the sealing cap 10 and the distal end 34 of the fluid passageway 26 and/or the distal end 32 of the needle 28. The maximum contact pressure of 50 MPa assures that the removal force required to overcome the frictional force between the sealing insert 62 and the outer surface of the distal tip 24 of the syringe 12 when removing the sealing cap 10 from the syringe 12 is low enough to allow easy, ergonomic removal of the sealing cap 10 from the syringe 12.

The radial thickness of the sidewall 58 of the housing 52 is approximately equal to the radial thickness of a sealing cap 10 constructed completely from the insert material that provides the desired contact pressure multiplied by the Young's modulus of the insert material divided by the Young's modulus of the housing material. For example, when the insert material is a thermoplastic elastomer with a Young's modulus of about 11 MPa that, when used as the sole material of the sealing cap 10, provides the desired contact pressure at a radial thickness of 1 mm, for a housing 52 of polypropylene (PP) with a Young's modulus of about 1300 MPa, the desired contact force can be achieved with a radial

$$0.1\,\text{mm}\,(100\,\mu\text{m})\left(1\,\text{mm}\,\times\,\frac{11\,\text{MPa}}{1300\,\text{MPa}}\,=0.1\,\text{mm}\right)$$

thickness of the sidewall 58 of the housing 52 of about . For housing materials having higher Young's moduli (stiffness), such as polycarbonate (PC), polyoxymethylene (POM), polybutylene terephthalate (PBT), or fiber-reinforced polymer, the ratio of the Young's modulus of the insert material to the Young's modulus of the housing material will be proportionally smaller and the radial thickness of the sidewall 58 of the housing 52 will be proportionally thinner.

The combination of the Young's modulus of the housing material and the radial thickness of the sidewall 58 of the housing 52 ensures that the sealing cap 10 will have the same contact force between the inner surface (insert) of the sealing cap 10 and the outer surface of the distal tip 24 of the syringe 12 as a cap made completely of the insert material and the combination of the insert material and the radial thickness of the sidewall 64 of the sealing insert 62 ensures that, based on the surface roughness of the distal tip 24 of the syringe 12, the sealing cap 10 will have the same frictional fit and sealing as a cap made completely of the insert material. Further, as discussed above, the substitution of the housing material having a higher Young's modulus for a portion of the material of the sealing cap 10 allows the overall radial thickness of the sidewall of the sealing cap 10 to be reduced as compared to a sealing cap made entirely of the lower Young's modulus material. Thus, the inventive sealing cap 10 provides sufficient contact force and sealing properties to provide an advantageous frictional fit and continuous seal between the sealing cap 10 and the outer surface of the distal tip 24 of the syringe 12 while reducing the overall size of the sealing cap 10, such that an overall diameter of the sealing cap 10 is equal to or less than the outer diameter of the syringe barrel 14.

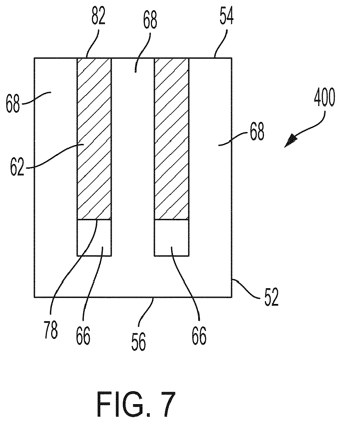

Any suitable geometry may be used for the housing 52 as long as the housing 52 provides the sealing cap 400 with the desired contact force and has an outer surface suitable for gripping by a user when removing the sealing cap 10. The housing may have an axially-symmetrical shape around a longitudinal axis and may be, for example, substantially cylindrical. As shown in FIG. 7, the housing 52 may include one or more recesses 66 where a portion or all of the radial thickness of the sidewall 58 of the housing 52 has been removed. If only a portion of the radial thickness of the sidewall 58 of the housing 52 is removed to form the recess 66, the recess 66 may be provided on the outer surface of the housing 52, the inner surface of the housing 52, or on both the outer surface and the inner surface of the housing 52. If a recess extends completely through the radial thickness of the sidewall 58 of the housing 52, the recess is completely covered by the sealing insert 62 to ensure that there is no opening between the exterior of the sealing cap 400 and the cavity 60 of the sealing cap 400 through which air and/or contaminants could enter the cavity 60.

As an example, a plurality of recesses 66 may be provided around a circumference of the housing 52, such that the material left between the recesses 66 is in the form of a plurality of legs 68 extending from a proximal portion of the housing 52 to a distal portion of the housing 52. The provision of the recesses 66 and legs 68 may act to reduce the overall stiffness of the housing 52 while still providing the desired contact force.

The geometry of the inner surface of the sealing insert 62 may have any shape that provides sufficient contact with the distal tip 24 of the syringe 12 to form a continuous seal between the sealing insert 62 and the outer surface of the distal tip 24 of the syringe 12 with the desired contact pressure. The geometry of the inner surface of the sealing insert 62 may substantially correspond to the geometry of the outer surface of the distal tip 24 of the syringe 12. For example, if the distal tip 24 of the syringe 12 has a substantially cylindrical outer surface, the sealing insert 62 may have a corresponding substantially cylindrical inner surface.

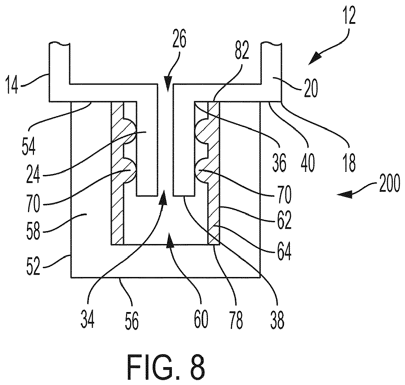

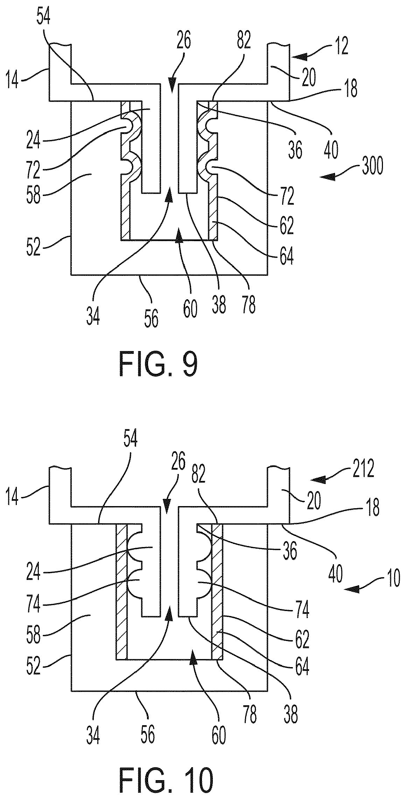

The sealing cap 200 (FIG. 8) may have a sealing insert 62 having one or more circumferential ribs 70 extending radially inward that contact the outer surface of the distal tip 24 of the syringe 12 to form the seal between the sealing insert 62 and the outer surface of the distal tip 24 of the syringe 12. The ribs 70 may be completely formed from the insert material. Alternatively, one or more circumferential ribs 72 may extend radially inward from the sidewall 58 of the housing 52 of the sealing cap 300 with the sealing insert 62 following the contours of the ribs 72 of the housing 52 (FIG. 9).

Circumferential ribs 74 that contact the insert to form a continuous seal may also be provided on the distal tip 24 of the syringe 212 (FIG. 10).

The sealing insert 62 may be attached to the housing 52 using any suitable connection including, but not limited to, co-injection, gluing, and deposition of the insert material onto the housing 52 or deposition of the housing material onto the sealing insert 62.

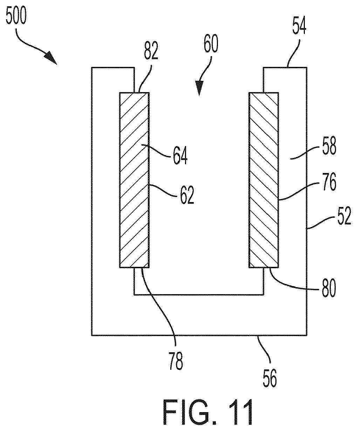

In another embodiment (FIG. 11), a recess 76 may be provided in an inner surface of the sidewall 58 of the housing 52 at the proximal end 54 of the housing 52, such that the sealing insert 62 is contained in the recess 76 with the distal end 78 of the sealing insert 62 resting on a distal ledge 80 created at a distal end of the recess 76. The proximal end 82 of the sealing insert 62 may also be contained by a proximal ledge 84 created at a proximal end of the recess 76. In another embodiment, a flange extending inwardly at the proximal end 54 of the housing 52 may contain the sealing insert 62 within the housing 52. When the proximal end 82 of the sealing insert 62 is restrained by the housing 52 via a restraint portion, such as a ledge or a flange, the housing 52 has an inner diameter that is greater than the inner diameter of the sealing insert 62 to allow the sealing cap 500 to be sufficiently inserted onto the distal tip 24 of the syringe 12 and create a frictional fit and a seal between the sealing insert 62 and the outer surface of the distal tip 24 of the syringe 12 without any interference caused by the restraint portion of the housing 52 contacting the distal tip 24 of the syringe 12.

Whereas particular aspects of this invention have been described above for purposes of illustration, it will be evident to those skilled in the art that numerous variations of the details of the present invention may be made without departing from the invention.

## Claims

1. A sealing cap for a syringe comprising a syringe barrel defining a reservoir for containing a medicament and a distal tip having a passageway in fluid communication with the reservoir, the sealing cap comprising:

   a housing having an open proximal end, a closed distal end, and a sidewall extending from the proximal end to the distal end and defining a cavity for receiving the distal tip; and
   a sealing insert covering at least a portion of an inner surface of the sidewall of the housing,
   the material properties of the housing are different from the material properties of the sealing insert,
   wherein an inner diameter of the sealing insert is less than an outer diameter of at least a portion of the distal tip, and
   the sealing cap is adapted to cover a distal end of the passageway and create a seal between the sealing insert and an outer surface of the distal tip to keep air and contaminants from entering the cavity of the sealing cap and/or the passageway.

2. The sealing cap of claim 1, wherein the sealing insert extends from the proximal end of the housing to the distal end of the housing, thereby covering the entire inner surface of the sidewall of the housing or the sealing insert covers a proximal portion of the inner surface of the sidewall of the housing leaving a distal portion of the inner surface of the housing uncovered.

3. The sealing cap of claim 1 or claim 2, wherein the sealing insert is a sleeve that is concentric with the sidewall of the housing.

4. The sealing cap of any of claims 1-3, wherein a material of the housing has a higher Young's modulus, higher strength, higher hardness, and/or lower elasticity than a material of the sealing insert.

5. The sealing cap of any of claims 1-4, wherein the sealing insert is made from a material selected from the group consisting of polyethylene (PE), polypropylene (PP), an elastomer, and rubber, and the housing is made from a material selected from the group consisting of polycarbonate (PC), polyoxymethylene (POM), polybutylene terephthalate (PBT), fiber-reinforced polymer, and metal.

6. The sealing cap of any of claims 1-5, wherein a radial thickness of a sidewall of the sealing insert is greater than a surface roughness of the outer surface of the distal tip.

7. The sealing cap of claim 6, wherein the radial thickness of the sidewall of the sealing insert is at least 20% thicker than the surface roughness of the outer surface of the distal tip.

8. The sealing cap of any of claims 1-7, wherein the sealing cap provides a contact pressure between the sealing insert and the outer surface of the distal tip of 0.1-50 MPa.

9. The sealing cap of claim 8, wherein a radial thickness of the sidewall of the sealing insert is minimized while being greater than a surface roughness of the outer surface of the distal tip, and a radial thickness of the sidewall of the housing is set to a thickness that provides the contact pressure.

10. The sealing cap of claim 8, wherein a radial thickness of the sidewall of the housing is approximately equal to a radial thickness of a cap constructed completely from a material of the insert that provides the contact pressure multiplied by a Young's modulus of the insert material divided by a Young's modulus of the housing material, such that the combination of the Young's modulus of the housing material and the radial thickness of the sidewall of the housing provides the sealing cap with the same contact force as the cap constructed completely of the insert material.

11. The sealing cap of any of claims 1-10, wherein the housing includes one or more recesses where a portion or all of a radial thickness of the sidewall of the housing has been removed.

**12.** The sealing cap of claim 11, wherein any recesses extending completely through the radial thickness of the sidewall of the housing are completely covered by the sealing insert.

**13.** The sealing cap of claim 11 or claim 12, wherein a plurality of recesses are provided around a circumference of the housing, such that material left between the recesses comprises a plurality of legs extending from a proximal portion of the housing to a distal portion of the housing.

**14.** The sealing cap of any of claims 1-13, wherein the sealing insert has one or more circumferential ribs extending radially inward.

**15.** The sealing cap of any of claims 1-14, wherein the housing has one or more circumferential ribs extending radially inward from the sidewall of the housing, and a contour of the sealing insert follows a contour of the housing.

**16.** The sealing cap of any of claims 1-15, wherein the sealing cap is adapted to cover a needle extending from the distal tip.

**17.** A syringe comprising:

a syringe barrel defining a reservoir for containing a medicament;
a distal tip having a passageway in fluid communication with the reservoir; and
a sealing cap according to claims 1-16,
wherein the sealing cap covers a distal end of the passageway and creates a seal between the sealing insert and an outer surface of the distal tip to keep air and contaminants from entering the cavity of the sealing cap and/or the passageway.

**18.** The syringe of claim 17, wherein the syringe further comprises a needle extending from the distal tip, and the sealing cap is adapted to cover the needle.

**19.** The syringe of claim 17 or claim 18, wherein circumferential ribs extend from an outer surface of the distal tip and contact the sealing insert.

**20.** The syringe of any of claims 17-19, wherein the outer surface of the distal tip is substantially cylindrical.

**21.** The syringe of any of claims 17-19, wherein the outer surface of the distal tip tapers such that an outer diameter of a proximal end of the distal tip adjacent a distal end of the syringe barrel is larger than an outer diameter of a distal end of the distal tip.

FIG. 1

FIG. 2

FIG. 3

FIG. 4

FIG. 5

FIG. 6

FIG. 7

FIG. 8

FIG. 9

FIG. 10

FIG. 11

Europäisches
Patentamt

European
Patent Office

Office européen
des brevets

# EUROPEAN SEARCH REPORT

Application Number

EP 23 17 0493

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 719 732 B2 (RUMPLER TECHNOLOGIES [FR]) 13 April 2004 (2004-04-13) * column 7 - column 8; figures 6,9 * ----- | 1-12, 14-21 | INV. A61M5/32 |
| X | US 2021/236744 A1 (MURRAY CHRISTOPHER J [US] ET AL) 5 August 2021 (2021-08-05) * paragraph [0060]; figures 14,11 * ----- | 1-3, 5-10, 13-20 | |
| X | US 5 980 495 A (HEINZ JOCHEN [DE] ET AL) 9 November 1999 (1999-11-09) * column 5; figures 1-5 * ----- | 1-3, 5-10, 16-18, 20,21 | |
| X | US 5 941 857 A (NGUYEN TUAN V [US] ET AL) 24 August 1999 (1999-08-24) * column 3 - column 4; figures 1-4 * ----- | 1-3, 5-10, 16-18, 20,21 | |

TECHNICAL FIELDS
SEARCHED        (IPC)

A61M

The present search report has been drawn up for all claims

| Place of search | Date of completion of the search | Examiner |
|---|---|---|
| Munich | 8 September 2023 | Hausmann, Alexander |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

**EP 23 17 0493**

This annex lists the patent family members relating to the patent documents cited in the above-mentioned European search report.
The members are as contained in the European Patent Office EDP file on
The European Patent Office is in no way liable for these particulars which are merely given for the purpose of information.

**08-09-2023**

| Patent document cited in search report | | Publication date | Patent family member(s) | | | Publication date |
|---|---|---|---|---|---|---|
| US 6719732 | B2 | 13-04-2004 | AT | 295193 | T | 15-05-2005 |
| | | | DE | 60110728 | T2 | 11-05-2006 |
| | | | DK | 1208861 | T3 | 29-08-2005 |
| | | | EP | 1208861 | A1 | 29-05-2002 |
| | | | ES | 2240375 | T3 | 16-10-2005 |
| | | | FR | 2816848 | A1 | 24-05-2002 |
| | | | PT | 1208861 | E | 30-09-2005 |
| | | | US | 2002062108 | A1 | 23-05-2002 |
| US 2021236744 | A1 | 05-08-2021 | NONE | | | |
| US 5980495 | A | 09-11-1999 | DE | 19717033 | A1 | 12-11-1998 |
| | | | EP | 0873758 | A2 | 28-10-1998 |
| | | | ES | 2186040 | T3 | 01-05-2003 |
| | | | JP | H10305098 | A | 17-11-1998 |
| | | | US | 5980495 | A | 09-11-1999 |
| US 5941857 | A | 24-08-1999 | CA | 2244982 | A1 | 12-03-1999 |
| | | | DE | 69805722 | T2 | 30-01-2003 |
| | | | EP | 0903157 | A2 | 24-03-1999 |
| | | | JP | 4298821 | B2 | 22-07-2009 |
| | | | JP | H11137687 | A | 25-05-1999 |
| | | | US | 5941857 | A | 24-08-1999 |

EPO FORM P0459

For more details about this annex : see Official Journal of the European Patent Office, No. 12/82